(19) Europäisches Patentamt
European Patent Office
Office européen des brevets

(11) Numéro de publication : **0 155 199**
**B1**

# (12) FASCICULE DE BREVET EUROPÉEN

(45) Date de publication du fascicule du brevet : **16.06.87**

(21) Numéro de dépôt : **85400168.2**

(22) Date de dépôt : **01.02.85**

**ERRATUM**

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE, COLONNE, LIGNE) | | LAUTET BERICHTIGT:<br>SHOULD READ :<br>DEVRAIT ETRE LU : |
|---|---|---|---|
| — point de fusion 57-99°C | 4 | 63 | — point de fusion 57-59°C |

Tag der Entscheidung über die Berichtigung
Date of decision on rectification:
Date de décision portant sur modification: 18.08.87

Ausgabe- und Veröffentlichungstag:
Issue and publication date:
Date d'edition et de publication: 24.02.88

Patbl.Nr
EPB no: 88/8
Bull. no:

(19) Europäisches Patentamt
European Patent Office
Office européen des brevets

(11) Numéro de publication : **0 155 199 B1**

(12) **FASCICULE DE BREVET EUROPÉEN**

(45) Date de publication du fascicule du brevet : **16.06.87**

(51) Int. Cl.[4] : **C 07 K 7/06**, A 61 K 37/02

(21) Numéro de dépôt : **85400168.2**

(22) Date de dépôt : **01.02.85**

(54) **Nouvel héxapeptide,son procédé d'obtention et les compositions pharmaceutiques qui le contiennent.**

(30) Priorité : **07.02.84 FR 8401816**

(43) Date de publication de la demande : **18.09.85 Bulletin 85/38**

(45) Mention de la délivrance du brevet : **16.06.87 Bulletin 87/25**

(84) Etats contractants désignés : **AT BE CH DE FR GB IT LI LU NL SE**

(56) Documents cités : **FR-A- 2 513 881**

(73) Titulaire : **INSTITUT NATIONAL DE LA SANTE ET DE LA RECHERCHE MEDICALE (INSERM)**
**101, rue de Tolbiac**
**F-75654 Paris Cedex 13 (FR)**

(72) Inventeur : **Jolles, Pierre**
**2 rue Jean-François Gerbillon**
**F-75006 Paris (FR)**
Inventeur : **Migliore-Samour, Danièle**
**64/66 avenue Charle Gide**
**F-94270 Le Kremlin-Bicêtre (FR)**
Inventeur : **Parker, Fabienne**
**32 avenue Paul Painlevé**
**F-94200 Saint-Maur-les-Fossés (FR)**
Inventeur : **Casaretto, Monika**
**Speicher Höhe 22**
**D-4050 Mönchengladbach (DE)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

EP 0 155 199 B1

## Description

La présente invention concerne l'hexapeptide de formule :

Val-Glu-Pro-Ile-Pro-Tyr (I)

son procédé d'obtention et les compositions qui le contiennent.

Dans la formule (I), Val représente la L-valine, Glu représente l'acide L-glutamique, Pro représente la L-proline, Ile représente la L-isoleucine et Tyr représente la L-tyrosine.

Dans le brevet européen 49 666 ont été décrites les substances désignées par MJH-24, MJH-63 et MJH-65 et leur préparation à partir de la caséine humaine délipidée traitée par la trypsine.

Les substances MJH-24, MJH-63 et MJH-65 qui sont des substances purifiées présentent des propriétés remarquables : ce sont des agents immunologiques qui favorisent la production d'anticorps et qui accélèrent le phénomène de phagocytose.

Il a maintenant été trouvé et c'est ce qui fait l'objet de la présente invention, que le fractionnement et la purification de la substance MJH-63 dans des conditions déterminées permettent d'isoler l'hexapeptide de formule (I) qui possède à un degré plus élevé les propriétés immunologiques de la substance MJH-63.

Pour obtenir l'hexapeptide de formule (I), il est nécessaire d'effectuer la succession des opérations suivantes :

a) filtration sur une membrane d'ultra-filtration de préférence une membrane Diaflo-Amicon UM-05 pour enlever la plus grande partie du chlorure de sodium,

b) filtration sur une colonne de Sephadex G-15 puis chromatographie sur une colonne de Dowex 50-X-4 pour éliminer totalement le chlorure de sodium, et le Tris

c) purification par chromatographie liquide à haute performance (HPLC).

Chaque étape de filtration et de purification est effectuée dans des conditions déterminées et est suivie en mesurant l'absorption à 220 et 280 nm des fractions recueillies.

La composition et la structure de l'hexapeptide de formule (I) sont déterminées :

— par hydrolyse totale par l'acide chlorhydrique 6N à 110 °C pendant 18 heures

— par dansylation, pour déterminer la nature de l'aminoacide N-terminal

— par dégradation automatique avec un séquenceur Beckmann, modèle 890 C.

L'hexapeptide de formule (I) peut être synthétisé par voie chimique par application des méthodes habituelles utilisées en chimie peptidique.

Par exemple, l'hexapeptide de formule (I) peut être préparé par application des méthodes d'allongement de chaîne par condensation d'aminoacides ou de peptides appropriés convenablement protégés suivie de l'élimination des groupements protecteurs.

Le dipeptide de formule :

Z-Pro-Tyr-OMe (II)

dans laquelle Z représente un groupement protecteur de la fonction amine, de préférence le groupement t.butyloxycarbonyle (Boc), après élimination du groupement protecteur Z, peut être condensé successivement avec l'isoleucine et la proline dont la fonction amine a été préalablement protégée pour donner le tétrapeptide de formule :

H-Pro-Ile-Pro-Tyr-OMe (III)

qui est lui-même condensé avec le dipeptide de formule :

Z-Val-Glu($Z_1$)-OH (IV)

dans laquelle Z est défini comme précédemment et $Z_1$ représente un groupement protecteur de la fonction acide, de préférence le groupement t.butyloxy ($OBu^t$), pour donner l'hexapeptide de formule :

Z-Val-Glu($Z_1$)-Pro-Ile-Pro-Tyr-OMe (V)

dont les groupements protecteurs des fonctions acides peuvent être éliminés par saponification en présence de nargase issue de Bacillus subtilis et dont le groupement protecteur de la fonction amine peut être éliminé, par exemple, au moyen d'acide trifluoroacétique dans un solvant organique tel que l'anisole, pour donner l'hexapeptide de formule (I).

Le dipeptide de formule (II) peut être obtenu par condensation de Z-Pro sur Tyr-OMe.

Le dipeptide de formule (IV) peut être obtenu par condensation de Z-Val sur Glu($Z_1$)-OH.

La condensation des aminoacides ou des peptides dont les fonctions amines sont convenablement protégées peut être effectuée en présence d'un agent de condensation tel que le dicyclohexylcarbodiimide ou bien en utilisant un anhydride mixte de l'aminoacide ou du peptide obtenu par exemple à partir

d'un halogénoformiate d'alcoyle ou de N-hydroxysuccinimide (ONSu).

L'hexapeptide de formule (I) ainsi obtenu peut être purifié par chromatographie sur un support convenable. Il est particulièrement avantageux d'effectuer une chromatographie sur une colonne échangeuse d'ions.

L'hexapeptide de formule (I) obtenu par synthèse chimique présente les mêmes propriétés que le produit obtenu par purification du produit naturel.

L'hexapeptide de formule (I) est un agent immunologique qui favorise la production d'anticorps et qui accélère le phénomène de phagocytose.

In vitro, il s'est montré particulièrement actif à des concentrations comprises entre 0,1 et 10 µg/cm³ dans le test de sécrétion d'anticorps (hémolytiques) anti-globules rouges de mouton par des cellules spléniques de souris immunisées in vivo et dans le test de phagocytose de globules rouges de moutons opsonisés par les macrophages péritonéaux de souris.

In vivo, l'hexapeptide de formule (I) fait preuve, chez la souris, d'une activité remarquable dès la dose de 0,05 mg/kg i. v. vis-à-vis de l'infection expérimentale à Klebsiella pneumoniae.

Les exemples suivants, donnés à titre non limitatif, illustrent l'obtention de l'hexapeptide selon l'invention.

Exemple 1

Le produit MJH-63, dont l'obtention est décrite dans l'exemple 3 du brevet européen 49 666, après filtration sur membrane Diaflo-Amicon UM-05, est filtré sur une colonne de Sephadex G-15 (hauteur 120 cm ; diamètre 1,2 cm) en éluant avec de l'acide acétique à 10 % avec une vitesse d'élution de 9 cm³/heure et en recueillant des fractions de 0,6 cm³.

Les fractions éluées entre 72 et 84 cm³ sont réunies puis chromatographiées sur une colonne de Dowex 50-X-4 (hauteur 14 cm ; diamètre 1,4 cm) en recueillant des fractions de 4,3 cm³.

L'élution est effectuée à la vitesse de 24 cm³/heure en utilisant successivement les éluants suivants :

1) acide chlorhydrique 0,05N (100 cm³)
2) eau (100 cm³)
3) ammoniaque 2N

Le diagramme d'élution est représenté par la figure 1.

Les fractions qui correspondent à l'élution entre 12 et 36 cm³ d'ammoniaque 2N sont réunies (fraction 3, MJH-72).

La poursuite de l'élution par le même solvant fournit une seconde fraction active (fraction 4, MJH-73).

La fraction 3, MJH-72, est purifiée par HPLC en phase « reverse » sur une colonne semi-préparative (colonne $C_{18}$-µ-bondapack, WATERS) dont la longueur est de 30 cm et le diamètre de 7,8 mm. On recueille des fractions de 0,5 cm³, la vitesse d'élution étant de 1 cm³/minute.

Au départ, la colonne est tamponnée par de l'acide phosphorique à 0,1 % (éluant A).

On prépare un éluant contenant de l'acide phosphorique (0,1 % en volume) et de l'acétonitrile (70 % en volume), dans l'eau (éluant B).

La fraction 3, MJH-72, est dissoute dans 250 µl d'acide phosphorique 0,1 %.

L'élution est effectuée en utilisant un gradient linéaire d'élution et en opérant selon le tableau suivant :

| Temps (minutes) | Eluant A | Eluant B |
|---|---|---|
| 0 | 100 | 0 |
| 10 | 100 | 0 |
| 110 | 50 | 50 |
| 140 | 0 | 100 |

Le diagramme d'élution est représenté par la figure 2.

L'élution est suivie en mesurant l'absorption à 280 nm et à 220 nm ainsi que par lecture à 490 nm de la fluorescence après réaction à la fluorescamine.

Le diagramme d'élution contient 49 zones dont les plus importantes quant à l'activité biologique sont les zones 41 à 47 (MJH-153) et la zone 49 (MJH-155) ; leurs temps de rétention sont respectivement 80 à 89 minutes et 91 à 93 minutes.

La fraction 49 (MJH-155) est à nouveau purifiée par HPLC sur une colonne de même nature que celle utilisée précédemment dont la longueur est de 30 cm et le diamètre de 3,9 mm. On recueille des fractions de 0,5 $cm^3$, la vitesse d'élution étant de 1 $cm^3$/minute.

On prépare les éluants suivants :

— éluant A : acide trifluoroacétique à 0,1 %
— éluant B : acide trifluoroacétique (0,1 % en volume) et acétonitrile (70 % en volume) dans l'eau

L'élution est effectuée en utilisant un gradient linéaire d'élution et en opérant selon le tableau suivant :

| Temps (minutes) | Eluant A | Eluant B |
|---|---|---|
| 0 | 80 | 20 |
| 15 | 80 | 20 |
| 30 | 70 | 30 |

Le diagramme d'élution est représenté par la figure 3.

Le diagramme d'élution contient 4 pics, le pic n° 4, élué entre 11,5 minutes et 13,5 minutes, concernant la fraction contenant l'hexapeptide de formule (I) pur.

La structure de l'hexapeptide est déterminée :

— par l'hydrolyse totale par l'acide chlorhydrique 6N à 100 °C pendant 18 heures qui montre la présence d'acide glutamique (Glu) = 1, de proline (Pro) = 2, de valine (Val) = 1, d'isoleucine (Ile) = 1 et de tyrosine (Tyr) = 1

— par dansylation, afin de déterminer la nature de l'aminoacide N-terminal, qui est la valine

— par analyse par le séquenceur Beckmann, modèle 890 C, programme Quadrol 0,1M avec détermination des différentes étapes grâce à la caractérisation des phénylthiohydantoïne-acides aminés (détermination par HPLC et révélation sur plaques).

Exemple 2

Dans ce qui suit, la pureté des produits est déterminée par chromatographie en couche mince en utilisant les systèmes de solvants suivants :

— chloroforme-méthanol-acide acétique (95-5-5 en volume) : Rf 1
— butanol-2-acide formique (90 %)-eau (75-13,5-11,5 en volumes) : Rf 2
— méthanol-acétate d'éthyle-eau (30-80-5 en volumes) : Rf 3
— n.butanol-acide acétique-pyridine-eau (15-3-10-12 en volumes) : Rf 4

L'analyse des aminoacides est effectuée après hydrolyse dans l'acide chlorhydrique 6N à 110 °C pendant 24 heures.

La synthèse de l'hexapeptide est réalisée de la manière suivante :

1) On dissout 6,45 g de Boc-Pro (30 m · moles) dans 20 $cm^3$ de diméthylformamide. Après refroidissement à — 15 °C on neutralise par addition de 3,3 $cm^3$ de N-méthylmorpholine (30 m · moles) puis on ajoute 3,93 $cm^3$ de chloroformiate d'isobutyle (30 m · moles).

Après 2 minutes, on ajoute une solution de 6,95 g de chlorhydrate de Tyr-OMe (30 m · moles) et de 3,3 $cm^3$ de N-méthylmorpholine (30 m · moles) dans 10 $cm^3$ de diméthylformamide. Le mélange réactionnel est agité pendant 1 heure à — 15 °C et pendant 2 heures à 20 °C. Le solvant est évaporé sous pression réduite. Le résidu obtenu est dissous dans l'acétate d'éthyle et la solution obtenue est lavée successivement par une solution d'acide citrique 1M, une solution de bicarbonate de sodium et une solution de chlorure de sodium.

La couche organique est séchée sur sulfate de sodium. Après élimination du solvant sous pression réduite, l'huile obtenue est reprise plusieurs fois par de l'éther éthylique pour donner 11,3 g de Boc-Pro-Tyr-OMe sous forme d'une mousse amorphe dont les caractéristiques sont les suivantes :

— point de fusion 57-99 °C
— $[\alpha]_D^{22}$ = — 34,2° (C = 1, méthanol)
— Rf 1 = 0,96 ; Rf 3 = 0,95

Le rendement est de 96 %.

2) On traite 6,4 g de Boc-Pro-Tyr-OMe par 10 $cm^3$ d'une solution d'acide trifluoroacétique dans l'anisole pendant 30 minutes à 20 °C. L'acide est éliminé sous pression réduite. Le résidu est dissous dans du diméthylformamide et la solution est neutralisée par addition de 1,8 $cm^3$ de N-méthylmorpholine (16 m · moles).

On fait réagir une solution de 3,5 g de Boc-Ile (16 m · moles) avec 3,8 g de hydroxy-1 benzotriazole (2,4 m · moles) et 3,7 g de N,N'-dicyclohexylcarbodiimide (18 m · moles) pendant 1 heure à 0 °C puis on ajoute la solution de Pro-Tyr-OMe dans 15 $cm^3$ de diméthylformamide préparée ci-dessus. Après agitation pendant 1 heure à 0 °C puis pendant 10 heures à 20 °C, le précipité de dicyclohexylurée est séparé par filtration et le filtrat est concentré. Le résidu obtenu est dissous dans l'acétate d'éthyle et la solution obtenue est lavée successivement par une solution d'acide citrique 1M, une solution de bicarbonate de sodium et une solution de chlorure de sodium. La couche organique est séchée sur sulfate de sodium. Après élimination du solvant, le produit brut est purifié par chromatographie sur une colonne de silicagel (hauteur 45 cm, diamètre 2,5 cm) en éluant avec de l'acétate d'éthyle et des mélanges acétate d'éthyle-hexane. On obtient 3,8 g de Boc-Ile-Pro-Tyr-OMe dont les caractéristiques sont les suivantes :

— point de fusion : 68 °C
— $[\alpha]_D^{22}$ = —55,9° (C = 1, méthanol)
— Rf 1 = 0,85 ; Rf 3 = 0,91
— analyse des aminoacides : Ile 1,00 (1) Pro 1,00 (1), Tyr 0,95 (1)

Le rendement est de 50,1 %.

3) On traite 2,4 g de Boc-Ile-Pro-Tyr-OMe (4,8 m · moles) par une solution d'acide trifluoroacétique dans l'anisole de la manière habituelle. L'acide est éliminé sous pression réduite. Le résidu est dissous dans du diméthylformamide et la solution obtenue est neutralisée par addition de 0,55 $cm^3$ de N-méthylmorpholine (4,8 m · moles).

On dissout 1,05 g de Boc-Pro (4,8 m · moles) dans 10 $cm^3$ de tétrahydrofuranne refroidi à —15 °C. On ajoute alors successivement 0,55 $cm^3$ de N-méthylmorpholine (4,8 m · moles) et 0,64 $cm^3$ de chloroformiate d'isobutyle (4,8 m · moles). Après 2 minutes, on ajoute la solution de Ile-Pro-Tyr-OMe dans 5 $cm^3$ de diméthylformamide préparée ci-dessus. Après agitation pendant 1 heure à —15 °C et pendant 2 heures à 20 °C, le mélange réactionnel est traité de la manière habituelle. On obtient ainsi 2,6 g de produit brut qui est purifié par chromatographie sur gel de silice en éluant avec un mélange acétate d'éthyle-éther de pétrole (1-1 en volumes) puis par un mélange acétate d'éthyle-méthanol (95-5 en volumes) et enfin par une nouvelle chromatographie en éluant avec un mélange acétate d'éthyle-méthanol (98-2 en volumes). On obtient ainsi 1,9 g de Boc-Pro-Ile-Pro-Tyr-OMe dont les caractéristiques sont les suivantes :

— point de fusion : 70-71 °C
— $[\alpha]_D^{22}$ = —100,4° (c = 1, méthanol)
— Rf 1 = 0,9 ; Rf 2 = 0,84
— analyse des aminoacides après 48 heures d'hydrolyse : Pro 1,99 (2) Ile 1,00 (1) Tyr 0,99 (1).

Le rendement est de 65,7 %.

4) On ajoute 4,5 g de Boc-Val-ONSu (15 m · moles) (ONSu = N-hydroxysuccinimide) à une solution de 3,65 g de H-Glu-($OBu^t$)-OH (18 m · moles) et de 3,0 g de bicarbonate de sodium (36 m · moles) dans 40 $cm^3$ d'un mélange dioxanne-eau (2-1 en volumes). Le mélange réactionnel est agité pendant 2 heures à 20 °C. Le dioxanne est éliminé sous pression réduite. Le résidu est extrait par l'acétate d'éthyle. La phase organique est lavée par une solution d'acide citrique 1M et par de l'eau. La purification est complétée par distribution à contre-courant à 160 passages dans un mélange n-butanol-eau. On obtient ainsi 4,8 g de Boc-Val-Glu($OBu^t$)-OH sous forme d'une huile. Le rendement est de 79,5 %.

5) On traite 1,0 g de Boc-Pro-Ile-Pro-Tyr-OMe (1,66 m · moles) une solution d'acide trifluoroacétique dans l'anisole de la manière habituelle. On neutralise par addition de 0,19 $cm^3$ de N-méthylmorpholine.

On dissout 0,8 g de Boc-Val-Glu($OBu^t$)-OH (1,9 m · mole) dans 5 $cm^3$ de tétrahydrofuranne refroidi à —15 °C. On ajoute successivement 0,21 $cm^3$ de N-méthylmorpholine (1,9 m · mole) et 0,25 $cm^3$ de chloroformiate d'isobutyle. Après 2 minutes, on ajoute la solution de Pro-Ile-Pro-Tyr-OMe dans 5 $cm^3$ de diméthylformamide préparée ci-dessus. Le mélange réactionnel est agité pendant 1 heure à —15 °C puis pendant 2 heures à 20 °C. Les solvants sont éliminés sous pression réduite. On obtient 1,2 g de produit brut qui est purifié par chromatographie sur gel de silice en éluant avec des mélanges acétate d'éthyle-éther de pétrole (1-1 ; 7-2 et 8-2 en volumes). La fraction principale est purifiée par chromatographie sur gel de silice en éluant avec du chlorure de méthylène à 3 % de méthanol (en volumes). On obtient ainsi 0,6 g de Boc-Val-Glu($OBu^t$)-Pro-Ile-Pro-Tyr-OMe dont les caractéristiques sont les suivantes :

— point de fusion : 100-102 °C
— $[\alpha]_D^{22}$ = —101,9° (c = 1, méthanol)

— Rf 1 = 0,92 ; Rf 4 = 0,4
— analyse des aminoacides : Val 1,00 (1), Glu 1,02 (1), Pro 1,84 (2), Ile 0,93 (1), Tyr 1,00 (1).

Le rendement est de 40,7 %.

6) La saponification de 100 mg de Boc-Val-Glu(OBu[t])-Pro-Ile-Pro-Tyr-OMe (1,1 m · mole) dissous dans 10 $cm^3$ de doxanne/0,1N en acétate de sodium (pH = 7,5) est catalysée par 1 mg de nargase issue de Bacillus subtilis. Après 3 heures, le mélange réactionnel est extrait à l'éther. La phase aqueuse est acidifiée à pH 3 puis extraite à l'acétate d'éthyle. On obtient ainsi 70,1 mg de produit brut qui est traité pendant 30 minutes par 5 $cm^3$ d'une solution d'acide trifluoroacétique dans l'anisole. L'acide est éliminé sous pression réduite et le résidu est trituré dans de l'éther éthylique, dissous dans l'acide acétique à 10 % puis désalifié par filtration sur Sephadex G25.

Le lyophylisat est purifié par chromatographie par échange d'ions sur une colonne de DEAE Sephadex A25 en éluant avec un gradient de bicarbonate d'ammonium 0,1-0,5M.

On obtient ainsi 48,3 mg de H-Val-Glu-Pro-Ile-Pro-Tyr-OH dont les caractéristiques sont les suivantes :

— Rf 4 = 0,66
— Analyse des aminoacides : Val 1,04 (1), Glu 1,00 (1), Ile 0,96 (1), Pro 1,87 (2), Tyr 0,97 (1)
— teneur en protéine : 98,2 %
— test de racémisation : Glu 2,44 % ; Tyr 1,2 % ; Ile 1,41 % ; Val 1 %.

La présente invention concerne également les compositions utilisables en thérapeutique humaine ou vétérinaire qui contiennent l'hexapeptide selon la présente invention en association avec un ou plusieurs diluants ou adjuvants compatibles et pharmaceutiquement acceptables.

Ces compositions peuvent être utilisées comme adjuvants de vaccins (par exemple vaccin antigrippal composé de sous-unités hémagglutinantes, vaccin antipolyomyélitique à virus inactivé, vaccin antimalarique) en injection simultanée avec l'antigène (viral, bactérien, parasitaire, fongique, tumoral) à l'égard duquel on souhaite augmenter la production d'anticorps ou la réactivité cellulaire spécifique.

Ces compositions pharmaceutiques peuvent être également employées comme immunostimulants non spécifiques en vue d'augmenter la résistance de l'hôte (homme ou animal domestique) vis-à-vis des infections ou en immunothérapie antitumorale.

En tant qu'adjuvant, le produit peut être administré soit en solution aqueuse, soit en émulsion huileuse, soit encore sous forme de liposomes avec l'antigène à l'égard duquel on souhaite obtenir une réponse immunitaire augmentée ou améliorée par la voie utilisée pour cet antigène et dans des proportions variant entre 0,01 et 10 fois la quantité d'antigène avec lequel ils sont mélangés avant d'être injectés.

Pour l'application comme immunostimulant non spécifique l'hexapeptide de formule (I) peut être utilisé par voie intraveineuse, intramusculaire, sous-cutanée, intranasale, éventuellement orale ou rectale, soit en solution aqueuse, soit en émulsion huileuse, soit encore sous forme de liposomes.

Dans ce cas, la dose d'hexapeptide selon l'invention est généralement comprise entre 0,01 et 25 mg/kg. En thérapeutique humaine, les doses journalières dépendent de l'effet recherché. Elles peuvent être comprises entre 0,1 et 10 mg pour un adulte.

Les compositions solides pour administration orale peuvent être des comprimés, des pilules, des poudres ou des granulés.

Comme compositions liquides pour administration orale, on peut utiliser des émulsions pharmaceutiquement acceptables, des solutions, des suspensions, des sirops ou des élixirs.

Les compositions pour administration parentérale ou intranasale peuvent être des solutions stériles aqueuses, des suspensions ou des émulsions.

La stérilisation peut se faire de plusieurs façons, par exemple à l'aide d'un filtre bactériologique ou en incorporant des agents stérilisants. Les compositions solides rendues stériles par irradiation (rayon β) peuvent être dissoutes dans de l'eau stérile ou tout autre milieu stérile injectable éventuellement au moment de l'emploi.

Les compositions pour administration rectale sont des suppositoires.

L'exemple suivant, donné à titre non limitatif, illustre une composition selon la présente invention.

Exemple

On prépare selon la technique habituelle une composition liquide administrable par voie intraveineuse ayant la composition suivante :

| | |
|---|---|
| — hexapeptide de formule (I) | 50 mg |
| — soluté injectable | 5 $cm^3$ |

**Revendications** (pour les Etats contractants : BE, CH, DE, FR, GB, IT, LI, LU, NL, SE)

1. L'hexapeptide de formule :

Val-Glu-Pro-Ile-Pro-Tyr

dans lequel les aminoacides sont sous forme L.

2. Procédé d'obtention de l'hexapeptide selon la revendication 1 à partir d'une fraction hydrosoluble de caractère acide, appelée MJH-63, obtenue par traitement de la caséine humaine délipidée et solubilisée par au moins une enzyme protéolytique puis fractionnement des produits hydrosolubles par filtration sur une colonne de Sephadex G-50 en éluant avec de l'acide acétique à 30 % des substances hydrosolubles éluées au niveau des produits de poids moléculaire moyen 2200 puis fractionnement de la substance hydrosoluble de poids moléculaire moyen 2200 sur une colonne de DEAE-Sephadex A-25 en éluant avec une solution tamponnée dont le pH va en décroissant, caractérisé en ce que la fraction MJH-63 est filtrée sur une membrane d'ultrafiltration Diaflo-Amicon UM-05 puis sur une colonne de Sephadex G-15 en éluant avec de l'acide acétique à 10 % puis sur une colonne de Dowex 50-X-4 en éluant successivement par de l'acide chlorhydrique 0,05N, de l'eau et de l'ammoniaque 2N puis purification de la fraction éluée par l'ammoniaque 2N par chromatographie liquide à haute performance et isolement de l'hexapeptide.

3. Procédé de préparation de l'hexapeptide selon la revendication 1 caractérisé en ce que l'on fait réagir le dipeptide de formule Boc-Val-Glu($OBu^t$)-OH sur le tétrapeptide de formule H-Pro-Ile-Pro-Tyr-OMe, obtenu par application des méthodes d'allongement de chaînes par condensations successives avec des aminoacides convenables à partir du dipeptide de formule Boc-Pro-Tyr-OMe, pour obtenir l'hexapeptide de formule Boc-Val-Glu($OBu^t$)-Pro-Ile-Pro-Tyr-OMe dont les groupements protecteurs des fonctions acides peuvent être éliminés par saponification catalysée par une enzyme extraite de Bacillus subtilis et dont le groupement protecteur de la fonction amine est éliminé au moyen d'acide trifluoroacétique pour obtenir l'hexapeptide selon la revendication 1.

4. Composition pharmaceutique caractérisée en ce qu'elle contient l'hexapeptide selon la revendication 1 en association avec un ou plusieurs diluants ou adjuvants compatibles et pharmaceutiquement acceptables.

**Revendications** (pour l'Etat contractant AT)

1. Procédé d'obtention de l'hexapeptide de formule :

Val-Glu-Pro-Ile-Pro-Tyr

dans lequel les aminoacides sont sous forme L à partir d'une fraction hydrosoluble de caractère acide, appelée MJH-63, obtenue par traitement de la caséine humaine délipidée et solubilisée par au moins une enzyme protéolytique puis fractionnement des produits hydrosolubles par filtration sur une colonne de Sephadex G-50 en éluant avec de l'acide acétique à 30 % des substances hydrosolubles éluées au niveau des produits de poids moléculaire moyen 2200 puis fractionnement de la substance hydrosoluble de poids moléculaire moyen 2200 sur une colonne de DEAE-Sephadex A-25 en éluant avec une solution tamponnée dont le pH va en décroissant, caractérisé en ce que la fraction MJH-63 est filtrée sur une membrane d'ultrafiltration Diaflo-Amicon UM-05 puis sur une colonne de Sephadex G-15 en éluant avec de l'acide acétique à 10 % puis sur une colonne de Dowex 50-X-4 en éluant successivement par de l'acide chlorhydrique 0,05N, de l'eau et de l'ammoniaque 2N puis purification de la fraction éluée par l'ammoniaque 2N par chromatographie liquide à haute performance et isolement de l'hexapeptide.

2. Procédé de préparation de l'hexapeptide de formule :

Val-Glu-Pro-Ile-Pro-Tyr

dans lequel les aminoacides sont sous forme L caractérisé en ce que l'on fait réagir le dipeptide de formule Boc-Val-Glu($OBu^t$)OH sur le tétrapeptide de formule H-Pro-Ile-Pro-Tyr-Ome, obtenu par application des méthodes d'allongement de chaînes par condensations successives avec des aminoacides convenables à partir du dipeptide de formule Boc-Pro-Tyr-OMe, pour obtenir l'hexapeptide de formule Boc-Val-Glu($OBu^t$)-Pro-Ile-Pro-Tyr-OMe dont les groupements protecteurs des fonctions acides peuvent être éliminés par saponification catalysée par une enzyme extraite de Bacillus subtilis et dont le groupement protecteur de la fonction amine est éliminé au moyen d'acide trifluoroacétique pour obtenir l'hexapeptide.

**Claims** (for the Contracting States : BE, CH, DE, FR, GB, IT, LI, LU, NL, SE)

1. The hexapeptide of formula :

Val-Glu-Pro-Ile-Pro-Tyr

in which the amino acids are in the L form.

2. Process for obtaining the hexapeptide according to Claim 1 from a water-soluble fraction of an acidic nature, called MJH-63, obtained by treatment of delipidized human casein and solubilized by means of at least one proteolytic enzyme followed by fractionation of the water-soluble products by filtering on a Sephadex G-50 column with elution with 30 % strength acetic acid of the water-soluble substances eluted in the region of the products of mean molecular weight of 2200 followed by fractionation of the water-soluble substance of mean molecular weight of 2200 on a DEAE-Sephadex A-25 column with elution with a buffered solution whose pH decreases progressively, characterized in that the MJH-63 fraction is filtered on a Diaflo-Amicon UM-05 ultrafiltration membrane and then on a Sephadex G-15 column with elution with 10 % strength acetic acid and then on a Dowex 50-X-4 column with successive elutions with 0.05N hydrochloric acid, water and 2N aqueous ammonia followed by purification of the fraction eluted by 2N aqueous ammonia by means of high-performance liquid chromatography and isolation of the hexapeptide.

3. Process for the preparation of the hexapeptide according to Claim 1, characterized in that the dipeptide of formula Boc-Val-Glu-(O-t-Bu)OH is reacted with the tetrapeptide of formula H-Pro-Ile-Pro-Tyr-OMe, obtained by the application of chain-lengthening methods by means of successive condensations with suitable amino acids from the dipeptide of formula Boc-Pro-Tyr-OMe, to obtain the hexapeptide of formula Boc-Val-Glu(O-t-Bu)-Pro-Ile-Pro-Tyr-OMe in which the groups protecting the acid functions may be removed by saponification catalysed by an enzyme extracted from Bacillus subtilis and in which the group protecting the amine function is removed by means of trifluoroacetic acid to obtain the hexapeptide according to Claim 1.

4. Pharmaceutical composition, characterized in that it contains the hexapeptide according to Claim 1, in combination with one or more compatible and pharmaceutically acceptable diluents or adjuvants.

**Claims** (for the Contracting State AT)

1. Process for obtaining the hexapeptide of formula :

Val-Glu-Pro-Ile-Pro-Tyr

in which the amino acids are in the L form, from a water-soluble fraction of an acidic nature, called MJH-63, obtained by treatment of delipidized human casein and solubilized by means of at least one proteolytic enzyme followed by fractionation of the water-soluble products by filtering on a Sephadex G-50 column with elution with 30 % strength acetic acid of the water-soluble substances eluted in the region of the products of mean molecular weight of 2200 followed by fractionation of the water-soluble substance of mean molecular weight of 2200 on a DEAE-Sephadex A-25 column with elution with a buffered solution whose pH decreases progressively, characterized in that the MJH-63 fraction is filtered on a Diaflo-Amicon UM-05 ultrafiltration membrane and then on a Sephadex G-15 column with elution with 10 % strength acetic acid and then on a Dowex 50-X-4 column with successive elutions with 0.05N hydrochloric acid, water and 2N aqueous ammonia followed by purification of the fraction eluted by 2N aqueous ammonia by means of high-performance liquid chromatography and isolation of the hexapeptide.

2. Process for the preparation of the hexapeptide of formula :

Val-Glu-Pro-Ile-Pro-Tyr

in which the amino acids are in the L form, characterized in that the dipeptide of formula Boc-Val-Glu(O-t-Bu)OH is reacted with the tetrapeptide of formula H-Pro-Ile-Pro-Tyr-OMe, obtained by the application of chain-lengthening methods by means of successive condensations with suitable amino acids from the dipeptide of formula Boc-Pro-Tyr-OMe, to obtain the hexapeptide of formula Boc-Val-Glu(O-t-Bu)-Pro-Ile-Pro-Tyr-OMe in which the groups protecting the acid functions may be removed by saponification catalysed by an enzyme extracted from Bacillus subtilis and in which the group protecting the amine function is removed by means of trifluoroacetic acid to obtain the hexapeptide.

**Patentansprüche** (für die Vertragsstaaten : BE, CH, DE, FR, GB, IT, LI, LU, NL, SE)

1. Hexapeptid der Formel

Val-Glu-Pro-Ile-Pro-Tyr

worin die Aminosäuren in der L-Form sind.

2. Verfahren zur Herstellung des Hexapeptids gemäß Anspruch 1, ausgehend von einer wasserlöslichen Fraktion mit saurem Charakter, genannt MJH-63, erhalten durch Behandlung von von Lipid befreitem und löslich gemachtem Casein durch mindestens ein proteolytisches Enzym, dann Fraktionierung der wasserlöslichen Produkte durch Filtrieren über eine Säule Sephadex G-50, wobei mit 30-%iger Essigsäure wasserlösliche Substanzen in Höhe der Produkte mit einem mittleren Molekulargewicht von 2200 eluiert werden, dann Fraktionieren der wasserlöslichen Substanz mit dem mittleren Molekulargewicht von 2200 auf einer Säule von DEAE-Sephadex A-25, indem mit einer Pufferlösung mit sinkendem pH eluiert wird, dadurch gekennzeichnet, daß die Fraktion MJH-63 über eine Ultrafiltrationsmembran Diaflo-Amicon UM-05, dann über eine Säule Sephadex G-15 filtriert wird, indem mit 10-%iger Essigsäure eluiert wird, dann über eine Säule von Dowex 50-X-4, wobei nacheinander mit 0,05N Salzsäure, Wasser und 2N-Ammoniak eluiert wird, anschließend Reinigung der eluierten Fraktion mit 2N-Ammoniak durch Hochleistungsflüssigkeitschromatographie und Isolierung des Hexapeptids.

3. Verfahren zur Herstellung des Hexapeptids gemäß Anspruch 1, dadurch gekennzeichnet, daß man das Dipeptid der Formel Boc-Val-Glu(OBu$^t$)-OH auf das Tetrapeptid der Formel H-Pro-Ile-Pro-Tyr-OMe einwirken läßt, das durch Anwendung von Kettenverlängerungsmethoden durch aufeinanderfolgende Kondensationen mit geeigneten Aminosäuren, ausgehend vom Dipeptid der Formel Boc-Pro-Tyr-OMe, erhalten wurde, zur Erzielung des Hexapeptids der Formel Boc-Val-Glu(OBu$^t$)-Pro-Ile-Pro-Tyr-OMe, dessen Schutzgruppen der Säurefunktionen durch Verseifung entfernt werden können, die durch ein Enzym, extrahiert aus Bacillus subtilis, katalysiert wird, und dessen Schutzgruppe der Aminfunktion mittels Trifluoressigsäure entfernt wird, um das Hexapeptid gemäß Anspruch 1 zu erhalten.

4. Pharmazeutische Zusammensetzung, dadurch gekennzeichnet, daß sie das Hexapeptid gemäß Anspruch 1 zusammen mit einem oder mehreren verträglichen und pharmazeutisch annehmbaren Verdünnungs- oder Hilfsmitteln enthält.

**Patentansprüche** (für den Vertragsstaat AT)

1. Verfahren zur Herstellung des Hexapeptids der Formel :

Val-Glu-Pro-Ile-Pro-Tyr

in welcher die Aminosäuren in L-Form sind, aus einer wasserlöslichen Fraktion sauren Charakters, genannt MJH-63, erhalten durch Behandlung von humanem lipidbefreitem und durch mindestens ein proteolytisches Enzym solubilisiertem Casein, anschließender Fraktionierung der wasserlöslichen Produkte durch Filtrieren an einer Sephadex G-50-Säule, wobei die wasserlöslichen Substanzen mit 30 %iger Essigsäure eluiert werden unter Eluierung von Produkten mit einem mittleren Molekulargewicht von 2200, und anschließender Fraktionierung der wasserlöslichen Substanz mit einem mittleren Molekulargewicht von 2200 an einer DEAE-Sephadex A-25-Säule, wobei mit einer gepufferten Lösung mit abnehmendem pH-Wert eluiert wird, dadurch gekennzeichnet, daß die Fraktion MJH-63 an einer Ultrafiltrationsmembran Diaflo-Amicon UM-05, dann an einer Sephadex G-15-Säule, wobei mit 10 %iger Essigsäure eluiert wird, und dann an einer Dowex 50-X-4-Säule filtriert wird, wobei nacheinander mit 0,05N Salzsäure, Wasser und 2N Ammoniak eluiert wird, worauf die mit 2N Ammoniak eluierte Fraktion mittels Hochleistungs-Flüssig-Chromatographie gereinigt wird und das Hexapeptid isoliert wird.

2. Verfahren zur Herstellung des Hexapeptids der Formel :

Val-Glu-Pro-Ile-Pro-Tyr

in welcher die Aminosäuren in L-Form sind, dadurch gekennzeichnet, daß man das Dipeptid der Formel Boc-Val-Glu(OBu$^t$)OH mit dem Tetrapeptid der Formel H-Pro-Ile-Pro-Tyr-OMe reagieren läßt, erhalten durch Anwendung der Methoden zur Kettenverlängerung durch aufeinanderfolgende Kondensationen mit geeigneten Aminosäuren ausgehend vom Dipeptid der Formel Boc-Pro-Tyr-OMe, um das Hexapeptid der Formel Boc-Val-Glu(OBu$^t$)-Pro-Ile-Pro-Tyr-OMe zu erhalten, deren Schutzgruppen mit Säurefunktionen durch katalytische Verseifung mit einem vom Bacillus subtilis stammenden Enzym abgespaltet wird, und deren Schutzgruppe mit Aminfunktion mit Trifluoressigsäure abgespaltet wird, um das Hexapeptid zu erhalten.

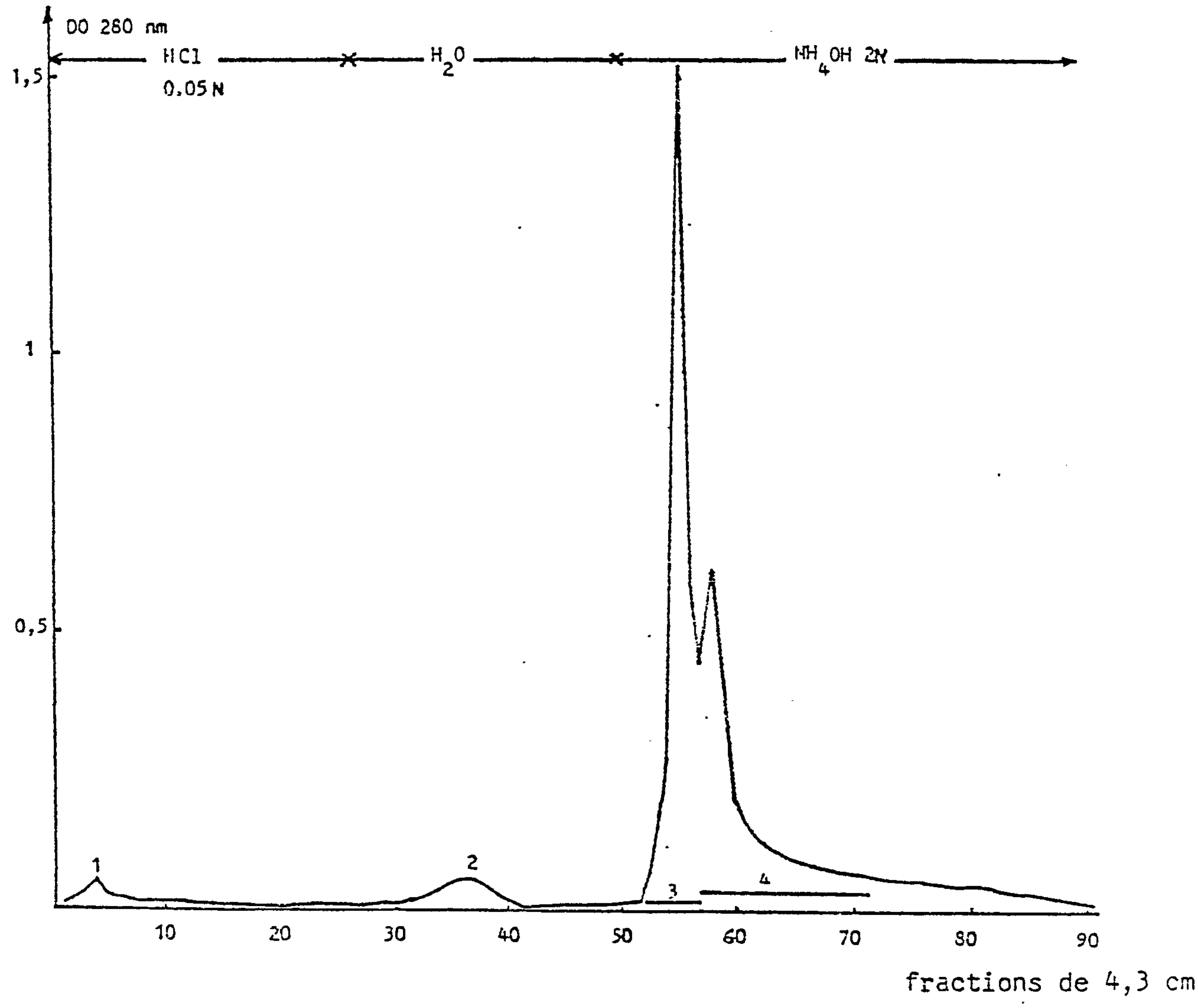
DO 260 nm
HCl
0,05 N
$H_2O$
$NH_4OH$ 2N
1,5
1
0,5
1
2
3
4
10
20
30
40
50
60
70
80
90
fractions de 4,3 cm

FIGURE 1

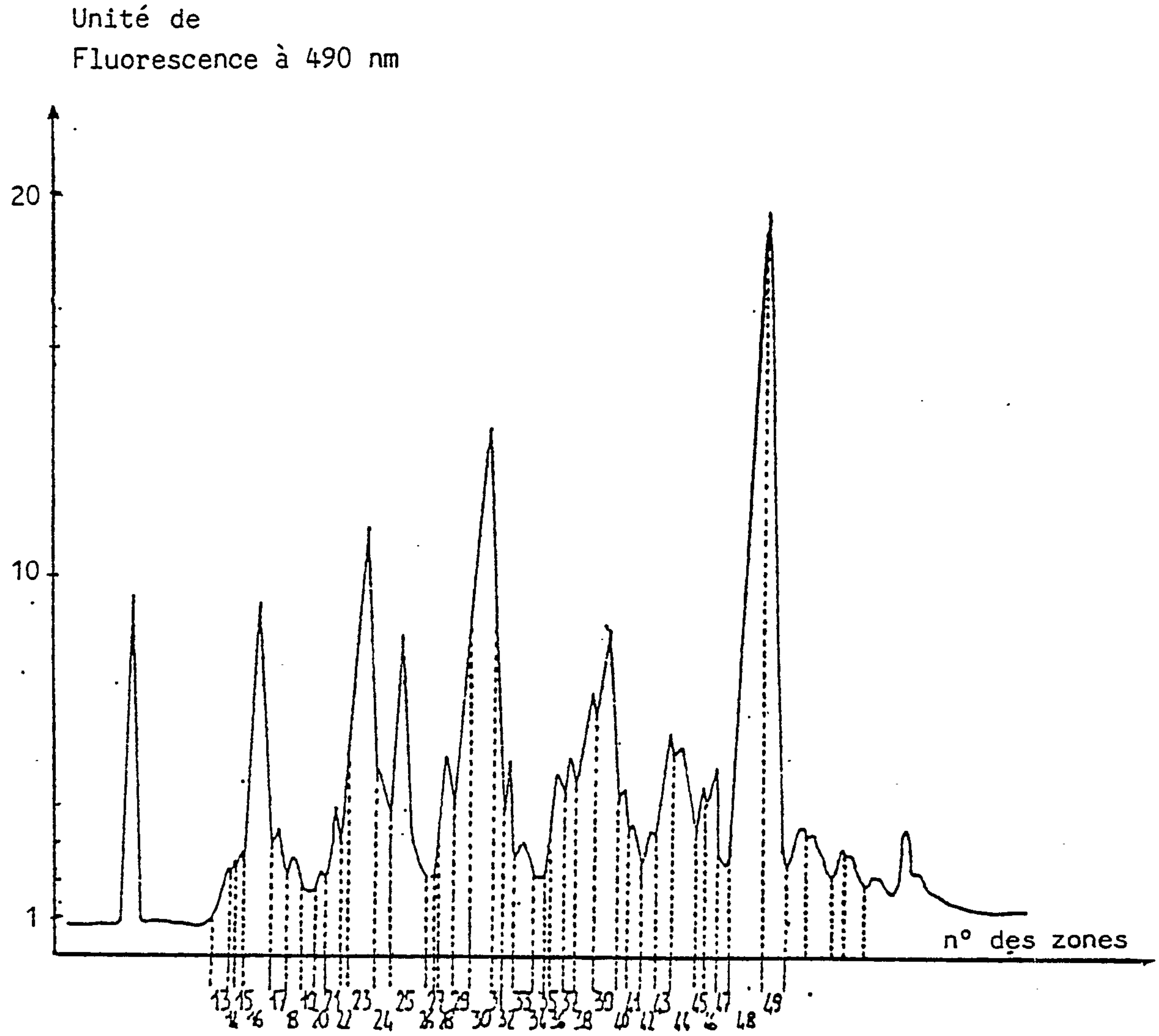


FIGURE 2

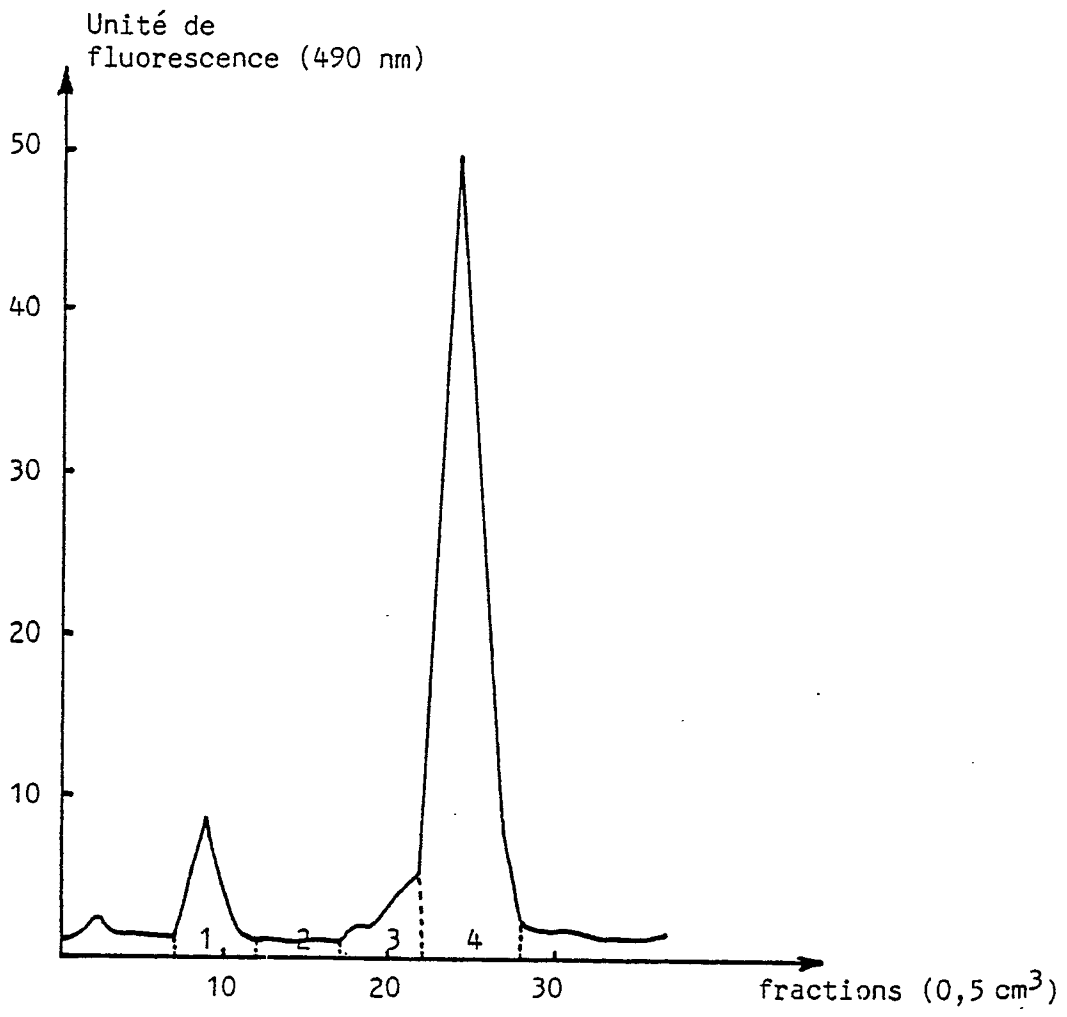
Unité de
fluorescence (490 nm)
50
40
30
20
10
1
2
3
4
10
20
30
fractions (0,5 cm3)

FIGURE 3